# EUROPÄISCHE PATENTANMELDUNG

(11) **EP 1 125 930 A1**
(43) Veröffentlichungstag der Anmeldung: **22.08.2001**
(21) Anmeldenummer: 01106243.7
(22) Anmeldetag: 22.05.1996
(51) Int. Cl.: C07D 241/04, B01J 31/22

(54) **Optisch aktive 2-Piperazincarbonsäurederivate und Verfahren zu ihrer Herstellung**

(62) Teilanmeldung aus: 96108171.8
(71) Anmelder: LONZA AG, CH-3945 Gampel/Wallis (CH)
(72) Erfinder: Fuchs, Rudolf, Dr., 3950 Sion (CH); Roduit, Jean-Paul, Dr., 3979 Grône (CH)
(74) Vertreter: Winter, Brandl, Fürniss, Hübner, Röss, Kaiser, Polte Partnerschaft

(57) **Zusammenfassung**

Optisch aktive 2-Piperazincarbonsäurederivate der allgemeinen Formel 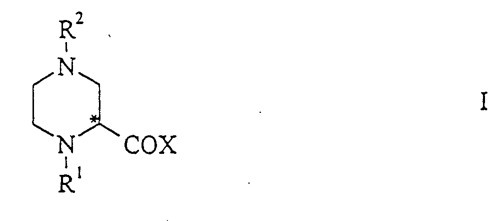 worin R¹ und R² u. a. Alkanoyl oder Perfluoralkanoyl bedeuten und X Alkoxy oder eine (substituierte) Aminogruppe ist, werden durch asymmetrische Hydrierung der entsprechenden 1,4,5,6-Tetrahydropyrazine unter Katalyse durch optisch aktive Rhodium-, Ruthenium- oder Iridiumkomplexe hergestellt. Die Verbindungen der Formel I sind Zwischenprodukte für die Herstellung von pharmazeutischen Wirkstoffen, beispielsweise von HIV-Protease-Inhibitoren.

## Beschreibung

Die vorliegende Erfindung betrifft ein Verfahren zur Herstellung von neuen optisch aktiven 2-Piperazincarbonsäurederivaten, insbesondere von Estern und Amiden der (*R*)- oder (*S*)-2-Piperazincarbonsäure, welche gegebenenfalls an den Ringstickstoffatomen Substituenten tragen und durch die allgemeine Formel 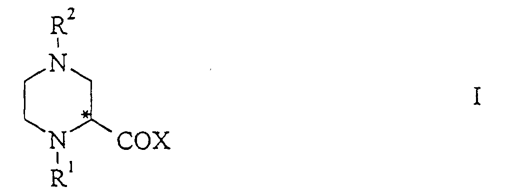 worin
R¹ und R² unabhängig voneinander jeweils Wasserstoff, C₁₋₄-Alkyl, gegebenenfalls substituiertes C₁₋₆-Alkanoyl, C₂₋₆-Perfluoralkanoyl, Aroyl, Arylalkyl, C₁₋₆-Alkoxycarbonyl, Aryloxycarbonyl, Carbamoyl oder eine Aminoschutzgruppe bedeuten und X eine Hydroxygruppe, eine C₁₋₆-Alkoxygruppe oder eine Gruppe der Formel -NR³R⁴ ist, worin wiederum
**(i)** R³ und R⁴ unabhängig voneinander Wasserstoff, C₁₋₆-Alkyl, C₃₋₆-Cycloalkyl, Aryl oder eine Aminoschutzgruppe bedeuten oder
**(ii)** R³ und R⁴ zusammen mit dem Stickstoffatom einen gegebenenfalls substituierten 5- oder 6-gliedrigen gesättigten heterocyclischen Ring bilden oder
**(iii)** R³ Wasserstoff und R⁴ eine Gruppe der Formel 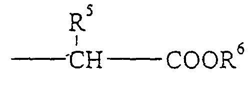 ist, worin R⁶ Wasserstoff, C₁₋₄-Alkyl oder Aryl und R⁵ Wasserstoff oder die Seitengruppe einer Aminosäure bedeuten,
repräsentiert werden, durch asymmetrische Hydrierung der entsprechenden 1,4,5,6-Tetrahydro-2-pyrazincarbonsäurederivate in Gegenwart von optisch aktiven Rhodium-, Ruthenium- und/oder Iridiumkomplexen.
Verbindungen dieser Gruppe sind wertvolle Zwischenprodukte, insbesondere für die Herstellung von pharmazeutischen Wirkstoffen. So sind beispielsweise einige bekannte Verbindungen mit X = NH-*t*-Bu und (*S*)-Konfiguration Bausteine für HIV-Protease-Inhibitoren (EP-A 541 168).

Die Erfindung betrifft weiterhin neue 1,4,5,6-Tetrahydro-2-pyrazincarbonsäurederivate als Ausgangsmaterial für die asymmetrische Hydrierung und ein Verfahren zu deren Herstellung.

Hier und im folgenden sind unter C₁₋ₙ-Alkyl jeweils geradkettige oder verzweigte primäre, sekundäre oder tertiäre Alkylgruppen mit 1 bis n Kohlenstoffatomen zu verstehen, also beispielsweise unter C₁₋₆-Alkyl Gruppen wie Methyl, Ethyl, Propyl, Butyl, Pentyl, Hexyl, Isopropyl, Isobutyl, *tert-Butyl,* Isopentyl und Neopentyl. Entsprechend sind unter C₁₋ₙ-Alkoxy und C₁₋ₙ-Alkoxycarbonyl die aus C₁₋ₙ-Alkyl und Sauerstoff bzw. Sauerstoff und Carbonyl zusammengesetzten Gruppen zu verstehen. Unter gegebenenfalls substituiertem C₁₋ₙ-Alkanoyl (C₁₋ₙ-Acyl) sind geradkettige oder verzweigte Alkanoylgruppen mit 1 bis n Kohlenstoffatomen (inclusive Carbonylkohlenstoff) zu verstehen, die noch Substituenten wie beispielsweise C₁₋₄-Alkoxygruppen tragen können, also beispielsweise unter C₁₋₆-Alkanoyl Gruppen wie Formyl, Acetyl, Methoxyacetyl, Propionyl, Butyryl, Isobutyryl, Valeryl oder Caproyl. Analog sind unter C₂₋ₙ-Perfluoralkanoyl die entsprechenden perfluorierten Acylgruppen wie beispielsweise Trifluoracetyl oder Pentafluorpropionyl zu verstehen. Unter Arylalkyl sind insbesondere Gruppen wie Benzyl oder Phenethyl zu verstehen, unter Aryl insbesondere Phenyl oder substituiertes Phenyl und unter Aroyl insbesondere Benzoyl oder substituiertes Benzoyl. Unter C₃₋₆-Cycloalkyl sind Cyclopropyl, Cyclobutyl, Cyclopentyl oder Cyclohexyl zu verstehen. Unter Aminoschutzgruppen sind die in der Peptidchemie üblichen Schutzgruppen wie beispielsweise Benzyloxycarbonyl (Z) oder *tert*-Butoxycarbonyl (Boc) zu verstehen. Der Ausdruck "gesättigter heterocyclischer Ring" umfasst auch Ringe mit mehreren, gegebenenfalls auch mehreren verschiedenen Heteroatomen wie beispielsweise Morpholin. Unter dem Begriff "Seitengruppen einer Aminosäure" sind die in der allgemeinen Aminosäureformel R-CH(NH₂)-COOH mit R bezeichneten Reste zu verstehen, insbesondere diejenigen der natürlichen Aminosäuren.

Bisher bekannte Synthesen von Verbindungen der Formel I gehen von der Stammverbindung (R¹ = R² = H, X = OH) aus, die durch klassische Racematspaltung in optisch aktiver Form aus dem Racemat erhalten werden kann (E. Felder et al., *Helv. Chim*. *Acta* **1960**, *43*, 888-896). Zunächst werden die Ringstickstoffatome geschützt und dann die freie Carboxylgruppe in das Amid übergeführt. Das Verfahren ist allenfalls für den Labormassstab geeignet und erfordert der Verwendung teurer Reagenzien. Aufgabe der vorliegenden Erfindung war daher, ein auch in technischem Massstab durchführbares Verfahren bereitzustellen, das ohne Racematspaltung auskommt.

Erfindungsgemäss wird diese Aufgabe durch das Verfahren gemäss Patentanspruch 1 gelöst.

Es wurde gefunden, dass sich die den Zielverbindungen entsprechenden 1,4,5,6-Tetrahydro-2-pyrazincarbonsäurederivate der allgemeinen Formel 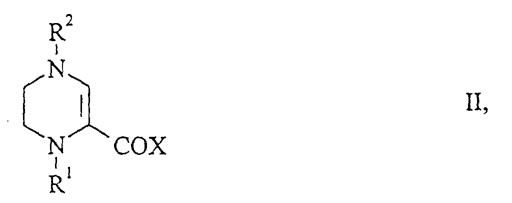 worin R¹, R² und X die oben genannten Bedeutungen haben, mit Wasserstoff in Gegenwart von katalytisch wirksamen optisch aktiven Rhodium-, Ruthenium- oder Iridiumkomplexen asymmetrisch zu den Zielverbindungen hydrieren lassen. Vorzugsweise werden solche 1,4,5,6-Tetrahydro-2-pyrazincarbonsäurederivate eingesetzt, in denen R¹ und/oder R² jeweils C₁₋₆-Alkanoyl, C₂₋₆-Perfluoralkanoyl, *tert*-Butoxycarbonyl ("Boc") oder Benzyloxycarbonyl ("Z") ist.
Ganz besonders bevorzugt werden solche 1,4,5,6-Tetrahydro-2-pyrazincarbonsäurederivate eingesetzt, in denen R¹ und/oder R² jeweils Formyl oder Trifluoracetyl ist. Diese Derivate haben den Vorteil, dass sich die Substituenten R¹ und R² gegebenenfalls selektiv abspalten lassen, nämlich die Formylgruppe unter sauren und die Trifluoracetylgruppe unter basischen Bedingungen.
Ebenfalls vorzugsweise werden als 1,4,5,6-Tetrahydro-2-pyrazincarbonsäurederivate die Amide eingesetzt, in denen X eine Gruppe der Formel -NR³R⁴ ist und R³ und R⁴ die obenstehenden Bedeutungen haben.
Besonders bevorzugt sind solche Amide, in denen R³ Wasserstoff und R⁴ eine C₁₋₆-Alkylgruppe, insbesondere eine *tert*-Butylgruppe ist.

Als katalytisch wirksamer optisch aktiver Rhodium-, Ruthenium- und/oder Iridiumkomplex wird vorzugsweise ein Rhodiumkomplex eingesetzt, der durch Reaktion eines Rh(I)-Komplexes mit einem optisch aktiven Metallocenylphosphin gebildet wird.

Als optisch aktive Metallocenylphosphine werden vorzugsweise Verbindungen der allgemeinen Formel 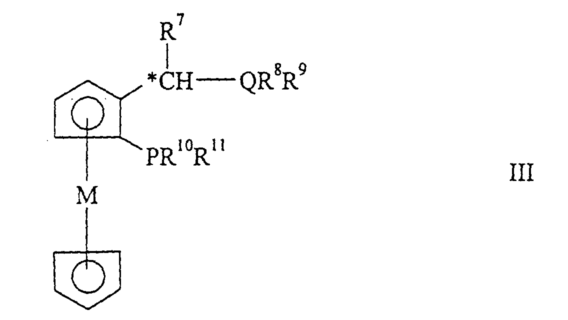 worin M Eisen(II) oder Ruthenium(II), Q Stickstoff oder Phosphor, R⁷ eine C₁₋₄-Alkylgruppe und R⁸ bis R¹¹ unabhängig voneinander C₁₋₈-Alkyl, C₅₋₈-Cycloalkyl, Phenyl oder substituiertes Phenyl bedeuten, eingesetzt.
Besonders bevorzugt sind die Metallocenylphosphine, in denen M Eisen ist, d. h. die Ferrocenylphosphine. Ebenfalls besonders bevorzugt sind die Metallocenylphosphine, in denen Q Phosphor ist, also Metallocenyldiphosphine.
Weiterhin besonders bevorzugt sind Metallocenylphosphine, in denen R⁷ Methyl ist und R⁸ und R⁹ gleich sind und *tert*-Butyl oder Cyclohexyl bedeuten. Zu diesen letztgenannten Metallocenylphosphinen zählen beispielsweise das 1-[1-(Di*-tert*-butylphosphino)ethyl]-2-(diphenylphosphino)ferrocen und das 1-[1-(Dicyclohexylphosphino)ethyl]-2-(diphenylphosphino)ferrocen. Die Herstellung dieser Verbindungen ist in EP-A 0 564 406 beschrieben. Weitere optisch aktive Metallocenylphosphine sind beispielsweise in T. Hayashi et al., *J*. *Am. Chem*. *Soc.* **1994**, *116*, 4221-4226, in EP-A 0 612 758 und in T. Hayashi et al., *Bull*. *Chem. Soc*. *Jpn*. **1980**, 53, 1138-1151 beschrieben.

Als Rh(I)-Komplexe, welche mit den optisch aktiven Metallocenylphosphinen zusammen die katalytisch wirksamen optisch aktiven Rhodiumkomplexe bilden, werden vorzugsweise neutrale zweikernige Komplexe der allgemeinen Formel

[Rh(L)A]₂ IV

eingesetzt. Hierbei steht L für ein C₄₋₁₂-Dien oder zwei C₂₋₁₂-Alkenmoleküle. A bedeutet Chlor, Brom oder Iod, vorzugsweise Chlor oder Brom.

Ebenfalls bevorzugt sind Rh(I)-Komplexe der allgemeinen Formel

[Rh(L)₂]⁺B⁻ V

worin L die oben genannte Bedeutung hat und B⁻ das Anion einer Sauerstoffsäure oder Komplexsäure ist. Unter Anionen von Sauerstoffsäuren sind beispielsweise Anionen wie ClO₄⁻, SO₃F⁻, CH₃SO₃⁻ oder CF₃SO₃⁻ zu verstehen, unter Anionen von Komplexsäuren solche wie beispielsweise BF₄⁻, PF₆⁻, AsF₆⁻, SbF₆⁻ oder SbCl₆⁻.

Besonders bevorzugt als Liganden L in den Rh(I)-Komplexen IV und V sind Diene, insbesondere Norbornadien und 1,5-Cyclooctadien.
Diese Komplexe sind bekannt, beispielsweise aus EP-A 0 302 021 oder US-A 5 011 995.

Die asymmetrische Hydrierung der 1,4,5,6-Tetrahydropyrazincarbonsäurederivate II wird vorteilhaft bei einer Temperatur von 20 bis 200 °C und einem Wasserstoffdruck von 1 bis 200 bar durchgeführt. Das molare Verhältnis Katalysator-Edukt liegt vorteilhaft bei 1:100 bis 1:5000, vorzugsweise bei 1:1000 bis 1:2000.
Als Lösungsmittel für die asymmetrische Hydrierung eignen sich beispielsweise Wasser, niedrige Alkohole wie Methanol, aromatische Kohlenwasserstoffe wie Toluol, Ketone wie Aceton oder Carbonsäureester wie Ethylacetat.

Die optisch aktiven 2-Piperazincarbonsäurederivate I, worin einer der beiden Substituenten R¹ und R² C₁₋₆-Alkanoyl oder C₂₋₆-Perfluoralkanoyl ist und der andere ebenso wie X die oben genannte Bedeutung hat, sind als solche, auch in Form ihrer Enantiomerengemische, ebenfalls Gegenstand der vorliegenden Erfindung. Unter Enantiomerengemischen sind hier insbesondere solche zu verstehen, in welchen ein Enantiomer gegenüber dem anderen angereichert ist.
Besonders bevorzugt sind diejenigen Verbindungen, in denen R¹ Trifluoracetyl und/oder R² Formyl ist.

Die 1,4,5,6-Tetrahydropyrazincarbonsäurederivate II sind neue Verbindungen und ebenfalls Gegenstand der vorliegenden Erfindung.
Sie können beispielsweise dadurch hergestellt werden, dass ein entsprechendes Pyrazincarbonsäurederivat der allgemeinen Formel 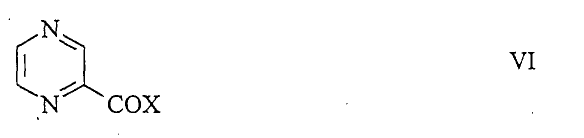 worin X die oben genannte Bedeutung hat, mit Wasserstoff an einem Palladiumkatalysator partiell zum 1,4,5,6-Tetrahydro-2-pyrazincarbonsäurederivat der allgemeinen Formel 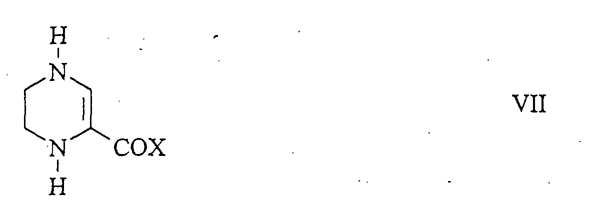 worin X die oben genannte Bedeutung hat, hydriert und anschliessend nach einer bekannten Methode, beispielsweise durch Acylierung mit einem Carbonsäureanhydrid, in die an den Ringstickstoffen N¹ und/oder N⁴ substituierte Verbindung übergeführt wird.

Verbindungen II, in denen X eine (substituierte) Aminogruppe ist, können auch ausgehend von 1,4,5,6-Tetrahydropyrazin-2-carbönitril hergestellt werden, beispielsweise durch eine Ritter-Reaktion. Die letztgenannte Verbindung ist nach einem in EP-A 0 175 364 beschriebenen Verfahren leicht zugänglich.

Die nachfolgenden Beispiele verdeutlichen die Durchführung des erfindungsgemässen Verfahrens und die Herstellung der erfindungsgemässen Verbindungen, ohne dass darin eine Einschränkung zu sehen ist.

### Beispiel 1

### Pyrazincarbonsäuremethylester

Zu 1200 ml Methanol wurden unter Argon bei 4-6 °C innerhalb von 1 h 106,6 g Thionylchlorid zugetropft. Bei 9 °C wurden 100,1 g Pyrazincarbonsäure zugegeben und das Gemisch 2 h auf 61 °C erwärmt, wobei die Säure vollständig in Lösung ging.
Nach dem Abkühlen auf Raumtemperatur wurde langsam eine Lösung von 145 g Natriumhydrogencarbonat in 1,4 1 Wasser zugegeben. Das Methanol wurde am Rotationsverdampfer bei 45 °C Badtemperatur und 50-120 mbar abdestilliert und der Rückstand dreimal mit Dichlormethan (400 ml, 100 ml, 100 ml) extrahiert. Durch Einengen der organischen Phase erhielt man 83,15 g Rohprodukt, das aus ca. 250 g Diisopropylether umkristallisiert wurde.
- Ausbeute:: 70,6 g, zuzüglich 10,28 g aus der Mutterlauge (total 72,5%)
- ¹H NMR (CDCl₃, 400 MHz): δ =: 4,08 (s, 3H);
8,75 (d, *J*= 0,5 Hz, 1H);
8,80 (d, *J*= 0,5 Hz, 1H);
9,30 (s, 1H).

### Beispiel 2

### 1,4,5,6-Tetrahydropyrazin-2-carbonsäuremethylester

In einem 500 ml-Autoklaven wurden in 100 ml Methylacetat 7,8 g Pyrazincarbonsäuremethylester (hergestellt nach Beispiel 1) und 1,5 g Palladium auf Aktivkohle (10% Pd) vorgelegt. Der Autoklav wurde zweimal mit Stickstoff und zweimal mit Wasserstoff gespült, indem das Gas bis 8 bar Druck aufgepresst und wieder entspannt wurde. Anschliessend wurde bei 20 °C und 10 bar Wasserstoffdruck unter Rühren 9 h hydriert. Danach wurde mit Stickstoff gespült, das Reaktionsgemisch über eine Filternutsche (0,45 µm Porenweite) filtriert und der Katalysator mit Methylacetat gewaschen. Durch Einengen wurde roher 1,4,5,6-Tetrahydro-2-pyrazincarbonsäuremethylester erhalten.
- ¹H NMR (CDCl₃, 400 MHz): δ =: 3,17-3,25 (m, 2H);
3,33-3,42 (m, 2H);
3,71 (s, 3H);
6,93 (br. s, 1H).

Das Produkt wurde ohne weitere Reinigung acyliert.

### Beispiel 3

### 1-Acetyl-1,4,5,6-tetrahydro-2-pyrazincarbonsäuremethylester

Zu 25,00 g (175,9 mmol) 1,4,5,6-Tetrahydro-2-pyrazincarbonsäuremethylester, 250 ml Tetrahydrofuran und 21,30 g (210,5 mmol) Triethylamin wurden bei 0 °C 15,10 g (192,5 mmol) Acetylchlorid innerhalb von 25 min zugetropft. Anschließend wurde auf Raumtemperatur erwärmt und nach 1 h 200 ml Wasser und 200 ml Essigsäureethylester zugegeben. Die Wasserphase wurde noch 3-4 mal mit insgesamt 500 ml Essigsäureethylester gewaschen und die vereinigten organischen Extrakte über Magnesiumsulfat getrocknet. Nach dem Abziehen des Lösungsmittels wurden 22,30 g (69%) der Titelverbindung als gelbes Öl erhalten. Umkristallisation aus Essigsäureethylester lieferte 1-Acetyl-1,4,5,6-tetrahydro-2-pyrazincarbonsäuremethylester als hellgelben, kristallinen Feststoff.
- Schmp.:: 136-139°C
- ¹H NMR (CDCl₃, 400 MHz): δ =: 2,10(s, 3H);
3,35 (br. s, 2H);
3,00-5,00 (br. m, 2H);
3,75 (s, 3H);
5,02 (br. s, 1H);
7,32 (d, 1H).

### Beispiel 4

### 1,4-Diacetyl-1,4,5,6-tetrahydro-2-pyrazincarbonsäuremethylester

In 200 g Essigsäureanhydrid wurden 10,1 g 1,4,5,6-Tetrahydro-2-pyrazincarbonsäuremethylester 2 h auf 135 °C erhitzt. Anschliessend wurde das überschüssige Essigsäureanhydrid bei 57 °C/55 mbar abdestilliert. Der Rückstand wurde mit 100 g Dichlormethan und 20 g Wasser versetzt. Die organische Phase wurde abgetrennt, mit 30 ml Wasser gewaschen und zur Trockne eingeengt. Als öliger Rückstand wurden 18,1 g Rohprodukt erhalten. Dieses wurde durch Mitteldruck-Säulenchromatographie (Säule 5×40 cm, Silicagel) mit Ethylacetat/Dichlormethan/Methanol (10:5:1) gereinigt.
- Ausbeute:: 11,6 g (85%)
- ¹H NMR (DMSO-d₆, 80 °C, 400 MHz): δ =: 1,98 (s, 3H);
2,25 (s, 3H);
3,60-3,70 (m, 1H);
3,70 (s, 3H);
7,60 (br. d, 1H).

### Beispiel 5

### 1-Acetyl-1,4,5,6-tetrahydro-2-pyrazincarbonsäure-tert-butylamid

Zu 100 ml (1,75 mol) Essigsäure wurden bei Raumtemperatur langsam 70 g (0,77 mol) Methansulfonsäure und anschliessend 20,0 g (184 mmol) 1,4,5,6-Tetrahydropyrazin-2-carbonitril gegeben. In diese Mischung wurde bei 25 °C 23,0 g (410 mmol) Isobuten eingeleitet und das Gemisch 5 h gerührt.
Anschliessend wurde mit 30%iger Natronlauge neutralisiert, wobei die Temperatur stets unter 30 °C gehalten wurde. Das auf pH 8-10 eingestellte Gemisch wurde dreimal mit je 200 ml Methylethylketon extrahiert. Die vereinigten organischen Phasen wurden über Magnesiumsulfat getrocknet und das Lösungsmittel abdestilliert. Der Rückstand (41,2 g) wurde in 80 ml Ethylacetat in der Hitze gelöst. Nach Abkühlung auf 20 °C wurden 500 ml Hexan zugegeben, weiter auf 0 °C abgekühlt, das ausgefallene Produkt nach 1 h abfiltriert und getrocknet.
- Ausbeute:: : 32,6 g (79%) hellbeiges Pulver
- Schmp.:: 151,3-152,6 °C
- ¹H NMR (CDCl₃, 400 MHz): δ =: 2,08 (s, 3H);
3,35 (br. s, 2H);
3,74 (s, 3H);
2,00-5,00 (br. m, 2H);
7,31 (d, *J* = 7,0 Hz, 1H).
- ¹³C NMR (CDCl₃, 100 MHz): δ =: 22,38 (CH₃);
37,28 (CH₂);
42,79 (CH₂);
51,15 (OCH₃);
103,63 (C=);
135,94 (CH=);
165,21 (COO);
171,85 (CON).

### Beispiel 6

### 1-Propionyl-1,4,5,6-tetrahydropyrazin-2-carbonsäure-tert-butylamid

Die Verbindung wurde analog zu Beispiel 5 unter Verwendung von Propionsäure anstelle von Essigsäure hergestellt.
- Ausbeute:: 75%
- Schmp:: 172,4-172,6 °C
- ¹H NMR (CDCl₃, 400 MHz):δ=: 1,09 (t, *J* = 7,3 Hz, 3H);
1,37 (s, 9H);
2,39 (q, *J*= 7,3 Hz, 2H);
3,28 (m, 2H);
3,57 (m, 2H);
5,41 (br. s, 1H);
5,49 (br. s, 1H);
7,09 (d, *J=* 6,3 Hz, 1H).
- ¹³C NMR (CDCl₃, 100 MHz): δ =: 9,44 (CH₃);
27,55 (CH₂);
29,12 (CH₃);
38,29 (CH₂);
42,19 (CH₂);
50,92 (C);
106,66 (C);
132,20 (CH);
165,21 (C=O);
177,20 (C=O).

### Beispiel 7

### 1-Isobutyryl-1,4,5,6-tetrahydropyrazin-2-carbonsäure-tert-butylamid

Die Verbindung wurde analog zu Beispiel 5 unter Verwendung von Isobuttersäure anstelle von Essigsäure hergestellt.
- Ausbeute:: 48%
- Schmp:: 180,0-184,4 °C
- ¹H NMR (CDCl₃, 400 MHz): δ =: 1,07 (d, *J*= 6,5 Hz, 6H);
1,37 (s, 9H);
2,82 (sept, *J=* 6,5 Hz, 1H);
3,27 (m, 2H);
3,6 (br. m, 2H);
4,94 (br. s, 1H);
5,48 (br. s, 1H);
7,12 (d, *J* = 6,3 Hz, 1H).
- ¹³C NMR (CDCl₃, 100 MHz): δ =: 19,36 (CH₃);
29,07 (CH₃);
32,50 (CH);
38,39 (CH₂);
42,42 (CH₂);
50,94 (C);
106,73 (C);
132,07 (CH);
165,21 (C=O);
180,85 (C=O).

### Beispiel 8

### 1,4-Diacetyl-1,4,5,6-tetrahydro-2-pyrazincarbonsäure-tert-butylamid

Zu 20,00 g (88,8 mmol) 1-Acetyl-1,4,5,6-tetrahydro-2-pyrazincarbonsäure-*tert*-butylamid wurden 16,7 g (163 mmol) Essigsäureanhydrid und 12,4 g (157 mmol) Pyridin gegeben und das Gemisch 4 h bei 25 °C gerührt. Anschliessend wurden 100 ml Wasser zugegeben und mit 30 ml Natronlauge (20%) auf pH 6 eingestellt. Dann wurde dreimal mit je 100 ml Methylethylketon ausgeschüttelt. Die vereinigten organischen Extrakte wurden über Magnesiumsulfat getrocknet, mit Toluol versetzt und eingeengt. Das stark schäumende Rohprodukt kristallisierte und 11,40 g (48%) der Titelverbindung wurden als leicht brauner Feststoff isoliert.
- Schmp.:: 138,0-139,2 °C
- ¹H NMR (CDCl₃, 400 MHz): δ =: 1,41 (s, 9H);
2,14 (s, 3H);
2,32 (s, 3H);
3,71-3,80 (m, 4H);
5,82 (br. s, 1H);
7,34 (s, 1H).
- ¹³C NMR (CDCl₃, 100 MHz): δ =: 21,30 (CH₃);
21,58 (CH₃);
28,76(CH₃);
39,44 (CH₂);
42,43 (CH₂);
51,76 (C);
116,61 (C);
123,36 (CH);
163,04 (C=0);
168,56 (C=O);
171,68 (C=O).

### Beispiel 9

### 4-Acetyl-1-propionyl-1,4,5,6-tetrahydro-2-pyrazincarbonsäure-tert-butylamid

168,7 g (0,705 mol) 1-Propionyl-1,4,5,6-tetrahydro-2-pyrazincarbonsäure-*tert*-butylamid wurden in 1,92 1 Butylacetat/Acetonitril-Gemisch (4:1) vorgelegt. Die Suspension wurde auf 50 °C geheizt und 68,2 g (0,812 mol) Natriumhydrogencarbonat wurden zugegeben. Eine Lösung von 57,0 g (0,726 mol) Acetylchlorid in 120 ml Butylacetat wurde innerhalb von 30 min zugetropft. Anschliessend wurde das Reaktionsgemisch noch 40 min bei 50 °C weitergerührt. Vorsichtig wurden 190 ml Wasser zugegeben, die wässerige Phase abgetrennt und nochmals bei 50 °C mit 200 ml Butylacetat extrahiert. Die vereinigten organischen Extrakte wurden auf 1100 ml aufkonzentriert und zur Kristallisation langsam auf 2 °C gekühlt. Der Feststoff wurde abfiltriert und mit 100 ml kaltem Butylacetat nachgewaschen. Umkristallisation aus 700 ml Butylacetat ergab 183,8 g (92,7%) der Titelverbindung als weisse Kristalle.
- Schmp.:: 133,5-134,6 °C
- ¹H NMR (CDCl₃, 400 MHz): δ =: 1,13 (t, *J* = 7,3 Hz, 3H);
1,40 (s, 9H);
2,32 (s, 3H);
2,38 (q, *J* = 7,3 Hz; 2H);
3,70 (m, 4H);
5,77 (br. s, 1H);
7,34 (s, 1H).
- ¹³C NMR (CDCl₃, 100 MHz): δ =: 9,27 (CH₃);
21,46 (CH₃);
27,88 (CH₂);
28,75 (CH₃);
39,88 (CH₂);
42,55 (CH₂);
51,72 (C);
116,49 (C);
123,43 (CH);
163,20 (C=O);
168,61 (C=O);
175,6 (C=O).

### Beispiel 10

### 1-Acetyl-4-benzoyl-1,4,5,6-tetrahydro-2-pyrazincarbonsäure-tert-butylamid

Analog zu Beispiel 9 wurde aus 1-Acetyl-1,4,5,6-tetrahydro-2-pyrazincarbonsäure-*tert*-butylamid und Benzoylchlorid die Titelverbindung als weisser kristalliner Feststoff erhalten.
- Schmp.:: 157,0-157,5 °C
- ¹H NMR (CDCl₃, 400 MHz): δ =: 1,37 (s, 9H);
2,17(s,3H);
3,78 (m, 2H);
3,89 (m, 2H);
5,74 (br. s, 1H);
7,50 (m, 6H).
- ¹³C NMR (CDCl₃, 100 MHz): δ =: 22,63 (CH₃);
28,74 (CH₃);
39,41 (CH₂);
44,05 (CH₂);
51,70 (C);
116,98 (C);
124,19 (C);
128,20 (CH);
128,87 (CH);
131,43 (CH);
133,33 (CH);
163,00 (C=O);
169,48 (C=O);
171,51 (C=O).

### Beispiel 11

### 1-Acetyl-4-methoxycarbonyl-1,4,5,6-tetrahydro-2-pyrazincarbonsäure-tert-butylamid

Analog zu Beispiel 9 wurde aus 1-Acetyl-1,4,5,6-tetrahydro-2-pyrazincarbonsäure-*tert*-butylamid und Chlorameisensäuremethylester die Titelverbindung als weisser kristalliner

Feststoff erhalten.
- Schmp.:: 134,0-135,3 °C
- ¹H NMR (CDCl₃, 400 MHz): δ =: 1,40 (s, 9H);
2,12 (s, 3H);
3,67 (m, 4H);
3,84 (s, 3H);
5,70 (br. s, 1H);
7,50 (br. s, 1H).
- ¹³C NMR (CDCl₃, 100 MHz): δ =: 22,54 (CH₃);
28,77 (CH₃);
39,07 (CH₂);
44,05 (CH₂);
51,63 (C);
53,86 (CH₃);
116,32 (C);
122,48 (CH);
152,93 (C=O);
163,26 (C=O);
171,72 (C=O).

### Beispiel 12

### 1-Acetyl-4-phenoxycarbonyl-1,4,5,6-tetrahydro-2-pyrazincarbonsäure-tert-butylamid

Analog dem Beispiel 9 wurde aus 1-Acetyl-1,4,5,6-tetrahydro-2-pyrazincarbonsäure*-tert*-butylamid und Chlorameisensäurephenylester die Titelverbindung als weisser kristalliner Feststoff erhalten.
- Schmp.:: 156,4-157,5 °C
- ¹H NMR (CDCl₃, 400 MHz): δ =: 1,41 (s, 9H);
2,16 (s, 3H);
3,79 (m, 4H);
5,76 (br. s, 1H);
7,13-7,41 (m, 6H).
- ¹³C NMR (CDCl₃, 100 MHz): δ =: 22,58 (CH₃);
28,74 (CH₃);
39,20 (CH₂);
44,27 (CH₂);
51,72 (C);
117,40 (C);
121,35 (CH);
126,23 (CH);
129,59 (CH);
150,59 (C);
150,9 (C=O);
163,10 (C=O);
171,60 (C=O).

### Beispiel 13

### 1,4-Dipropionyl-1,4,5,6-tetrahydro-2-pyrazincarbonsäure-tert-butylamid

Analog zu Beispiel 9 wurde aus 1-Propionyl-1,4,5,6-tetrahydro-2-pyrazincarbonsäure-*tert*-butylamid und Propionylchlorid die Titelverbindung als weisser kristalliner Feststoff erhalten.
- Schmp.:: 131,0-132,8 °C
- ¹H NMR (CDCl₃, 400 MHz): δ =: 1,13 (t, *J* = 7,3 Hz, 3H);
1,20 (t, *J* = 7,3 Hz, 3H);
1,40 (s, 9H);
2,38 (q, *J* = 7,3 Hz, 2H);
2,60 (q, *J*= 7,3 Hz, 2H);
3,70 (m, 4H);
5,77 (br. s, 1H);
7,39 (s, 1H).
- ¹³C NMR (CDCl₃, 100 MHz): δ =: 8,81 (CH₃);
9,27(CH₃);
26,68 (CH₂);
27,85 (CH₂);
28,76 (CH₃);
39,88 (CH₂);
42,67 (CH₂);
51,69 (C);
116,29 (C);
122,86 (CH);
163,29 (C=O);
171,93 (C=O);
175,49 (C=O).

### Beispiel 14

### 4-Acetyl-1-isobutyryl-1,4,5,6-tetrahydro-2-pyrazincarbonsäure-tert-butylamid

Analog zu Beispiel 9 wurde aus 1-Isobutyryl-1,4,5,6-tetrahydro-2-pyrazincarbonsäure-*tert*-butylamid und Acetylchlorid die Titelverbindung als weisser kristalliner Feststoff erhalten.
- Schmp.:: 116,3-117,2 °C
- ¹H NMR (CDCl₃, 400 MHz): δ =: 1,10 (d, *J* = 6,6 Hz, 6H);
1,40 (s, 9H);
2,33 (s, 3H);
2,72 (m, 1H);
3,70 (m, 4H);
5,80 (br. s, 1H);
7,37 (s, 1H).
- ¹³C NMR (CDCl₃, 100 MHz): δ =: 19,33 (CH₃);
21,46 (CH₃);
28,67 (CH₃);
33,04 (CH);
39,81 (CH₂);
42,67 (CH₂);
51,65 (C);
116,17 (C);
123,49 (CH);
163,32 (C=O);
168,60 (C=O);
179,11 (C=O).

### Beispiel 15

### 1-Acetyl-4-methoxyacetyl-1,4,5,6-tetrahydro-2-pyrazincarbonsäure-tert-butylamid

Analog zu Beispiel 9 wurde aus 1-Acetyl-1,4,5,6-tetrahydro-2-pyrazincarbonsäure-*tert*-butylamid und Methoxyacetylchlorid die Titelverbindung als weisser kristalliner Feststoff erhalten.
- Schmp.:: 109,6-110,7 °C
- ¹H NMR (CDCl₃, 400 MHz): δ =: 1,41 (s, 9H);
2,14 (s, 3H);
3,46 (s, 3H);
3,73 (m, 4H);
4,32 (s, 2H);
5,73 (br. s, 1H);
7,32 (s, 1H).
- ¹³C NMR (CDCl₃, 100 MHz): δ =: 22,60 (CH₃);
28,76 (CH₃);
39,33 (CH₂);
42,67 (CH₂);
51,82 (C);
59,51 (CH₃);
71,31 (CH₂);
117,34 (C);
121,88 (CH);
162,89 (C=O);
167,26 (C=O);
171,62 (C=O);

### Beispiel 16

### 1-Propionyl-4-trifluoracetyl-1,4,5,6-tetrahydro-2-pyrazincarbonsäure-tert-butylamid

Analog zu Beispiel 9 wurde aus 1-Propionyl-1,4,5,6-tetrahydro-2-pyrarincarbon-säure-*tert*-butylamid und Trifluoracetylchlorid die Titelverbindung als weisser kristalliner Feststoff erhalten.
- Schmp.:: 132,6-133,3 °C
- ¹HNMR (C₂D₂Cl₄, 400 MHz, 100°C): δ =: 1,13 (t, *J* = 7, Hz, 3H);
1,38 (s, 9H);
2,35 (q, *J*= 7,3 Hz, 2H);
3,80 (m. 4H);
5,66 (br. s, 1H);
7,28 (br. s, 1H).
- ¹³C NMR(C₂D₂Cl₄, 100 MHz, 100°C): δ =: 9,15 (CH₃);
27,86 (CH₂);
28,71 (CH₃);
40,10 (CH₂);
44,90 (CH₂);
51,88 (C);
115,87 (q, ¹*J*_{CF} = 286 Hz, CF₃);
117,75 (CH);
122,05 (C);
154,47 (q, ²*J*_{CF} = 38,7 Hz, COCF₃);
162,17 (CONH);
173,96 (COEt).

### Beispiel 17

### 4-Formyl-1-propionyl-1,4,5,6-tetrahydro-2-pyrazincarbonsäure-tert-butylamid

### Verfahren A: Formylierung mit in situ generiertem Formylchlorid

Zu einer Suspension von 70,00 g (0,293 mol) 1-Propionyl-1,4,5,6-tetrahydro-2-pyrazincarbonsäure-*tert*-butylamid in 350 ml Butylacetat wurde bei 20 °C 70,00 g (1,521 mol) Ameisensäure zugegeben. Zu der erhaltenen gelblichen Lösung wurde innerhalb von 4,5 h eine Lösung aus 63 g Thionylchlorid in 140 ml Butylacetat zugetropft. Die Temperatur wurde dabei unter 25 °C gehalten. Die Reaktionslösung wurde mit 50 ml 5%iger Natriumhydrogencarbonatlösung gewaschen und die wässerige Phase mit 100 ml Dichlormethan nachgewaschen. Beide organische Phasen wurden zusammengegeben und nach dem Entfernen des Lösungsmittels der Rückstand aus Butylacetat umkristallisiert. 71,2 g (82,7%) der Titelverbindung wurden als weisser kristalliner Feststoff erhalten.

### Verfahren B: Formylierung mit Acetyl-formyl-anhydrid (analog Lit.: J.C. Sheehan, D.-D. Yang, J. Am. Chem. Soc. 1958, 80, 1154.)

Zu einer Lösung von 31,2 g (0,13 mol) 1-Propionyl-1,4,5,6-tetrahydro-2-pyrazincarbonsäure-tert-butylamid in 40,0 g (0,87 mol) Ameisensäure wurde bei 30 °C ein Gemisch aus 20,0 g (0,196 mol) Essigsäureanhydrid und 18,00 g (0,391 mol) Ameisensäure innerhalb von 30 min zugetropft. Das Reaktionsgemisch wurde noch 20 min bei 30 °C gerührt. Dann wurde 50 ml Wasser zugegeben und die klare Lösung 50 min bei 40 °C gehalten. Das Produkt wurde extrahiert und aus *n*-Butylacetat kristallisiert. 30,75 g (88%) der Titelverbindung wurden als weisser kristalliner Feststoff erhalten.
- Schmp.:: 132,1-133,5 °C
- ¹H NMR (CDCl₃, 400 MHz): δ =: 1,14 (t, *J* = 7,3 Hz, 3H);
1,40 (s, 9H);
2,39 (q, *J*= 7,3 Hz, 2H);
3,66 (m, 2H);
3,74 (m, 2H);
5,75 (br. s, 1H);
7,22 (s, 1H);
8,41 (s, 1H).
- ¹³C NMR (CDCl₃, 100 MHz): δ =: 9,25 (CH₃);
27,89 (CH₂);
28,67 (CH₃);
39,25 (CH₂);
45,06 (CH₂);
51,79 (C);
118,3 (C);
122,9 (CH);
161,01 (C=O);
162,79 (C=O);
175,4 (C=O).

### Beispiel 18

### 1-Acetyl-4-formyl-1,4,5,6-tetrahydro-2-pyrazincarbonsäure-tert-butylamid

Die Verbindung wurde analog zu Bsp. 17 (Verf. B) aus 1-Acetyl-1,4,5,6-tetrahydro-2-pyrazincarbonsäure-*tert*-butylamid und Ac₂O/HCOOH als weisser kristalliner Feststoff erhalten.
- Schmp.:: 131,0-132,9 °C
- ¹H NMR(CDCl₃, 400 MHz): δ =: 1,41 (s, 9H);
2,15 (s, 3H);
3,70 (m, 4H);
5,75 (br. s, 1H);
7,21 (s, 1H);
8,41 (s, 1H).
- ¹³C NMR (CDCl₃, 100 MHz): δ =: 22,62 (CH₃);
28,73 (CH₃);
38,65 (CH₂);
44,99 (CH₂);
51,85 (C);
118,27 (C);
122,90 (CH);
160,94 (C=O);
162,63 (C=O);
171,7 (C=O).

### Beispiel 19

### (S)-1,4-Diacetyl-2-piperazincarbonsäure-tert-butylamid

Ein Autoklav wurde unter Sauerstoffausschluss mit 10 g (37 mmol) 1,4-Diacetyl-1,4,5,6-tetrahydro-2-pyrazincarbonsäure-*tert*-butylamid, 20,3 mg (37 µmol) 1-[1(*R*)-(Di-*tert*-butylphosphino)ethyl]-2(*S*)-(diphenylphosphino)ferrocen und 9,2 mg (20 µmol) Bicyclo[2.2.1]hepta-2,5-dienrhodium(I)chlorid-Dimer versehen. Nach Spülen mit Argon wurde 80 ml sauerstofffreies Methanol zugegeben. Bei einem Anfangsdruck von 50 bar und einer Temperatur von 110°C wurde 20 h hydriert. Der Autoklav wurde entspannt und mit Stickstoff gespült. Das Lösungsmittel des Reaktionsgemisches wurde vollständig abdestilliert und man erhielt so 10,50 g (95%) des Titelproduktes. Die GC-Analyse zeigte einen Umsatz von 85%. Die Enantioselektivität der Hydrierung wurde nach Abspaltung der Schutzgruppen mit Salzsäure durch GC-Analyse zu 90,5% ee bestimmt.

### Beispiel 20

### (S)-1,4-Diacetyl-2-piperazincarbonsäure-tert-butylamid

Ein Autoklav wurde unter Sauerstoffausschluss mit 8,00 g (29,9 mmol) 1,4-Diacetyl-1,4,5,6-tetrahydro-2-pyrazincarbonsäure*-tert*-butylamid, 10,0 mg (18 µmol) 1-[1(*R*)-(Di-*tert*-butylphosphino)ethyl]-2(S)-(diphenylphosphino)ferrocen und 6,0 mg (16 µmol) Bis(bicyclo[2.2.1]-hepta-2,5-dien)-rhodium(I)-tetrafluorborat versehen. Nach Spülen mit Argon wurden 80 ml sauerstofffreies Methanol zugegeben. Bei einem Anfangsdruck von 50 bar und einer Temperatur von 110°C wurde 20 h hydriert. Nach Aufarbeitung des Reaktionsgemisches analog zu Beispiel 19 erhielt man 10,50 g (75%) des Titelproduktes. Die GC-Analyse zeigte einen Umsatz von 97%. Die Enantioselektivität der Hydrierung wurde nach Abspaltung der Schutzgruppen mit Salzsäure durch GC-Analyse zu 90,7% ee bestimmt.
- Schmp.:: 139,9-150,6 °C
- ¹H NMR (CDCl₃, 400 MHz): δ =: 1,31; 1,33 (2 s, 9H),
2,10; 2,12; 2,15; 2,17; 2,19; 2,22; 2,26 (7 s, 6H);
2,77-2,83 (m, 1H);
3,18-3,28 (m, 2H);
3,70-3,73 (m, 1H);
4,41-4,49 (m, 2H);
5,04 (br. s, 1H);
5,95 (br. s, 1H).

### Beispiel 21

### (S)-4-Formyl-1-propionyl-2-piperazincarbonsäure-tert-butylamid

Ein Autoklav wurde unter Sauerstoffausschluss mit 16,1 g (60,2 mmol) 4-Formyl-1-propionyl-1,4,5,6-tetrahydro-2-pyrazincarbonsäure-*tert*-butylamid, 19,5 mg (35,9 µmol) 1-[1(*R*)-(Di-*tert*-butylphosphino)ethyl]-2(*S*)-(diphenylphosphino)ferrocen und 11,1 mg (29,6 µmol) Bis(bicyclo[2.2.1]hepta-2,5-dien)-rhodium(I)-tetrafluorborat versehen. Nach Spülen mit Argon wurde 80 ml sauerstofffreies Methanol zugegeben. Bei einem Anfangsdruck von 12 bar und einer Temperatur von 90°C wurde 8 h hydriert. Der Autoklav wurde entspannt und mit Stickstoff gespült. Das Lösungsmittel des Reaktionsgemisches wurde vollständig abdestilliert und man erhielt 17,00 g (94%) des Titelproduktes. Die GC-Analyse zeigte einen Umsatz von 99%. Die Enantioselektivität der Hydrierung wurde nach Abspaltung der Schutzgruppen mit Salzsäure durch GC-Analyse zu 96,5% ee bestimmt.
- Schmp.:: 132,1-134,3 °C
- ¹H NMR (CDCl₃, 400 MHz): δ =: 1,21 (t, *J* = 7,3 Hz, 3H);
1,30; 1,31 (2 s, 9H);
2,46 (q, *J=* 7,3 Hz, 2H);
2,75-2,82 (m, 1H);
3,12-3,18 (m, 2H);
3,60-3,78 (m, 1H);
4,21-4,38 (m, 2H);
5,07 (br. s, 1H);
5,92 (br. s, 1H);
8,10; 8,18 (2 s, 1H).

### Beispiele 22-41

Analog zu Beispiel 19 wurden verschiedene Tetrahydropyrazin-Derivate II in Anwesenheit von 1-[1(*R*)-(Di-*tert*-butylphosphino)ethyl]-2(*S*)-(diphenylphosphino)ferrocen und [Rh(cod)Cl]₂ (A) oder Rh(nbd)₂BF₄ (B) in Methanol hydriert (cod = 1,5-Cyclooctadien, nbd = Bicyclo[2.2.1]heptadien). Die Ergebnisse sind in der folgenden Tabelle 1 zusammengestellt. Angegeben ist jeweils das molare Verhältnis Edukt : Rhodium, die Substituenten X, R¹ und R², die Reaktionsbedingungen, der verwendete Katalysator, der Umsatz und der Enantiomerenüberschuss (ee). Als Produkt wurde jeweils bevorzugt das (*S*)-Enantiomer gebildet.

**Tabelle 1**

| Edukt:Rh | X/R¹/R² | Temp [°C]/ Zeit [h] | Druck [bar] | Katalysator | Umsatz [%] | ee [%] |
|---|---|---|---|---|---|---|
| 500 | NH-*t*-Bu/Ac/Ac | 90/4 | 50 | B | 94 | 93,7 |
| 1000 | NH-*t*-Bu/EtCO/EtCO | 90/22 | 50 | B | 91 | 87,5 |
| 1000 | NH-*t*-Bu/EtCO/Ac | 90/22 | 10 | B | 94 | 91 |
| 1000 | NH-*t*-Bu/Ac/COPh | 90/21 | 50 | B | 83 | 89,9 |
| 475 | NH-*t*-Bu/Me₂CHCO/Ac | 90/21 | 50 | B | 96,9 | 86,3 |
| 1000 | NH-*t*-Bu/Ac/COOMe | 90/20 | 50 | B | 97 | 88,3 |
| 480 | NH-*t*-Bu/Ac/CHO | 90/1 | 50 | B | 99,8 | 96,9 |
| 487 | NH-t-Bu/EtCO/CHO | 90/2 | 50 | B | 99,7 | 96,5 |
| 482 | NH-*t*-Bu/EtCO/CHO | 70/6 | 12 | B | 99,8 | 96,8 |
| 475 | NH-*t*-Bu/CHO/CHO | 70/6 | 12 | B | 72,4 | 91,7 |
| 400 | NH-*t*-Bu/EtCO/COCF₃ | 90/17 | 50 | B | 95 | 88,2 |
| 400 | NH-*t*-Bu/Ac/MeOCH₂CO | 70/6 | 12 | B | 99 | 84,5 |
| 250 | NH-*t*-Bu/Ac/H | 70/20 | 50 | A | 87 | 58 |
| 500 | NH-*t*-Bu/EtCO/H | 90/24 | 50 | A | 78 | 52 |
| 500 | NH-*t*-Bu/COCHMe₂/H | 90/23 | 50 | B | 74 | 41 |
| 500 | MH-*t*-Bu/COCH₂OMe/H | 90/23 | 50 | B | 39 | 56 |
| 25 | OMe/H/H | 50/20 | 50 | A | 87 | 40 |
| 250 | OMe/Ac/H | 70/20 | 50 | A | 87 | 58 |
| 500 | OMe/Ac/H | 70/22 | 50 | A | 59 | 37,8 |
| 245 | OMe/Ac/Ac | 50/20 | 50 | A | 74 | 95 |

### Beispiel 42

### 1-Trifluoracetyl-1,4,5,6-tetrahydropyrazin-2-carbonsäure-tert-butylamid

In einem 500 ml Kolben wurden 50 ml Trifluoressigsäure unter Argon vorgelegt. Bei 21 °C wurden 12,5 g Methansulfonsäure zugetropft und dann portionsweise 20 g 2-Cyan-1,4,5,6-tetrahydropyrazin-Methansulfonsäuresalz (97 mmol) zugegeben, wobei eine leichte Exothermie beobachtet wurde. Anschliessend wurden innerhalb 1 h bei 20 °C 10 g (178 mmol) Isobuten eingeleitet. Das Reaktionsgemisch wurde noch 2 h bei 20 °C gerührt, dann bei dieser Temperatur tropfenweise mit 13,3 g (111 mmol) Thionylchlorid versetzt und noch weitere 20 h gerührt. Dann wurden 250 ml Dichlormethan und portionsweise 25 g Natriumacetat zugegeben. Nach Filtration der rohen Lösung durch Celite® wurden 30 ml Wasser zugegeben und die Phasen getrennt. Die organische Phase wurde zur Trockne eingeengt. Das zurückbleibende Rohprodukt (27,88 g) wurde mit Ethylacetat/Methanol (4:1) an 300 g Kieselgel chromatographiert.
- Ausbeute (GC):: 29,2%.
- Schmp.:: 158-160 °C (aus *n*-Butylacetat).
- ¹H NMR (CDCl₃, 400 MHz) δ =: 1,35 (s, 9H);
3,43 (br. s, 2H);
3,74 (br. s, 2H);
5,32 (br. s, 1H);
5,53 (br. s, 1H);
7,06 (d, *J* = 6 Hz, 1H).
- ¹³C NMR (CDCl₃, 100 MHz) δ =: 28,9;
42,3;
42,8;
51,2;
105,9;
116,3 (¹*J*_{CF} = 288 Hz);
133,2;
154,5 (²*J*_{CF} = 35 Hz);
163,7.
- MS: *m/z =*: 57 (100%), 279 (M⁺, 6,2%).

### Beispiel 43

### 4-Formyl-1-trifluoracetyl-1,4,5,6-tetrahydropyrazin-2-carbonsäure-tert-butylamid

Zu einer Lösung von 96,94 g (347,1 mmol) 1-Trifluoracetyl-1,4,5,6-tetrahydropyrazin-2-carbonsäure-*tert*-butylamid in 465,9 g (10,1 mol) Ameisensäure wurden innerhalb von 40 min bei 20 °C 49,60 g (416,9 mmol) Thionylchlorid zugegeben. Nach 2 h Reaktionsdauer bei 20 °C wurde das Gemisch bei 60 °C und 80 mbar am Rotationsverdampfer eingeengt und der braunschwarze Rückstand mit 400 ml Ethylacetat und 400 ml Wasser aufgenommen. Die wässrige Phase wurde noch zweimal mit je 250 ml Ethylacetat gewaschen und die vereinigten organischen Extrakte über 50,1 g Natriumsulfat getrocknet. Nach Filtration wurde die Lösung am Rotationsverdampfer eingeengt und der dickflüssige Rückstand mit 250 ml Diisopropylether gerührt. Der ausgefallene Feststoff wurde abfiltriert, zweimal mit je 100 ml Diisopropylether gewaschen und getrocknet.
- Ausbeute:: 96,76 g (91%) farblose Kristalle
- Schmp.:: 158,4-160,3 °C

NMR-Daten: Im ¹H- und im ¹³C NMR ist ein jeweils ein doppelter Signalsatz sichtbar. Die Signale des Hauptkonformeren sind mit ^{*} markiert, soweit eine Zuordnung möglich war.
- ¹H NMR (CDCl₃, 400 MHz) δ =: 8,40 (s, 1H)*;
8,14 (s, 1H);
7,41 (s, 1H);
7,14 (s, 1H)*;
5,65 (br. s, 1H);
5,55 (br. s, 1H)^{*};
3,93-3,80 (m, 2×4H);
1,40 (s, 9H);
1,39 (s, 9H)^{*}.
- ¹³C NMR (CDCl₃, 100 MHz) δ =: 161,69;
161,14;
160,49;
159,61;
122,76;
119,76;
117,42;
115,84 (¹*J*_{CF} = 288 Hz);
52,04;
52,00;
44,77;
41,83;
41,11;
28,62.

### Beispiel 44

### (S)-4-Formyl-1-(trifluoracetyl)piperazin-2-carbonsäure-tert-butylamid

In einem 160 ml Autoklaven wurden 20,6 g (67,0 mmol) 4-Formyl-1-trifluoracetyl-1,4,5,6-tetrahydropyrazin-2-carbonsäure-*tert*-butylamid, 26,2 mg (64,5 µmol) Bis(1,5-cyclooctadien)rhodium(I)tetrafluorborat und 41,8 mg (77 µmol) 1-[1(*R*)-(Di-*tert*-butylphosphino)ethyl]-2(*S*)-(diphenylphosphino)ferrocen unter Argon vorgelegt (S/C = 1000). Man gab 70 ml Aceton (entgast) zu und hydrierte 11 h bei 100°C unter 13-10 bar Wasserstoffdruck. Nach dem Abdestillieren des Lösungsmittel wurden 20,5 g (99%) der Titelverbindung als kristalliner Feststoff erhalten.
- ee =: 96,4%
- Schmp.:: 172,0-173,0°C
- ¹H NMR (DMSO-d₆, 400 MHz, 70 °C) δ =: 8,06 und 8,02 (2 s, 1H);
7,63 (br. "d", 1H);
4,90-2,95 (m, 7H);
1,28 und 1,23 (2 s, 9H).
- ¹³C NMR (DMSO-d₆, 100 MHz, 70 °C) Ausgewählte Signale: δ=: 166,94;
166,70;
161,05;
160,94;
156,22 (q, ²*J*_{CF} = 35 Hz, COCF₃);
116,32 (q, ¹*J*_{CF} = 288 Hz, CF₃).

### Beispiel 45

### (S)-1-(Trifluoracetyl)piperazin-2-carbonsäure-tert-butylamid-Schwefelsäuresalz (1:1)

In einem 50 ml Autoklaven wurden 2,50 g (8,95 mmol) 1-Trifluoracetyl-1,4,5,6-tetrahydropyrazin-2-carbonsäure-*tert*-butylamid, 7,5 mg (18 µmol) Bis(1,5-cyclooctadien)rhodium(I)-tetrafluorborat und 11,5 mg (21 µmol) 1-[1(R)-(Di-*tert*-butylphosphino)ethyl]-2(*S*)-(diphenylphosphino)ferrocen unter Argon vorgelegt (Edukt: Katalysator = 486). Dann gab man 0,89 g (9,08 mmol) Schwefelsäure gelöst in 20 ml entgastem Tetrahy drofuran zu. Anschliessend wurde 18 h bei 20°C unter 13-10 bar Wasserstoffdruck hydriert. Nach dem Abdestillieren des Lösungsmittels erhielt man 3,42 g (>99 %) der Titelverbindung als weisses Pulver.
- ee =: 79,5%
- ¹H NMR(DMSO-d₆, 400 MHz) δ=: 9,60-8,30 (m, 2H);
7,88 (br. s, 1H);
4,95-3,05 (m, 7H);
1,32 (s, 9H).
- ¹³C NMR (DMSO-d₆, 100 MHz) Ausgewählte Signale: δ =: 166,94 (CONH);
115,90 (q, ¹*J*_{CF} = 288 Hz, CF₃).

### Beispiel 46

### (S)-1-(Trifluoracetyl)piperazin-2-carbonsäure-tert-butylamid

In einem 50 ml Autoklaven wurden 2,50 g (8,95 mmol) 1-Trifluoracetyl-1,4,5,6-tetrahydropyrazin-2-carbonsäure-*tert*-butylamid, 7,5 mg (18 µmol) Bis(1,5-cyclooctadien)rhodium(I)-tetrafluorborat und 11,5 mg (21 µmol) 1-[1(*R*)-(Di-*tert*-butylphosphino)ethyl]-2(*S*)-(diphenylphosphino)ferrocen unter Argon vorgelegt (Edukt: Katalysator = 486). Man gab 20 ml entgastes Ethylacetat hinzu und hydrierte 18 h bei 90°C unter 13-10 bar Wasserstoffdruck. Nach dem Abdestillieren des Lösungsmittels erhielt man 2,50 g der rohen Titelverbindung als hellgelbes Pulver.
- ee =: 47,2%
- ¹H NMR (CDCl₃, 400 MHz) (Nur die Signale des Hauptisomeren sind angegeben.) δ =: 5,88 (br. s, 1H);
4,68-4,65 ("d", 1H);
3,85-3,78 ("d", 1H);
3,54-3,49 ("d", 1H);
3,41-3,32 ("dt", 1H);
3,11-3,06 ("d", 1H);
2,90-2,78 (m, 2H);
2,07 (s, 1H);
1,34 (s, 9H).

### Beispiel 47

### (S)-1-(Trifluoracetyl)piperazin-2-carbonsäure-tert-butylamid-BF₃-Addukt

In einem 50 ml Autoklaven wurden 2,50 g (8,95 mmol) 1-Trifluoracetyl-1,4,5,6-tetrahydropyrazin-2-carbonsäure-*tert*-butylamid, 7,5 mg (18 µmol) Bis(1,5-cyclooctadien)rhodium(I)-tetrafluorborat und 11,5 mg (21 µmol) 1-[1(*R*)-(Di-*tert*-butylphosphino)ethyl]-2(*S*)-(diphenylphosphino)ferrocen unter Argon vorgelegt (Edukt: Katalysator = 488). Man gab eine Lösung von 0,71 g (6,83 mmol) Bortrifluorid-dihydrat in 20 ml entgastem Tetrahydrofuran zu. Anschliessend wurde 18 h bei 90°C unter 13-10 bar Wasserstoffdruck hydriert. Nach dem Abdestillieren des Lösungsmittels erhielt man 3,09 g der rohen Titelverbindung als weisses Pulver.
ee = 51,6%

### Beispiel 48

### (S)-4-Formylpiperazin-2-carbonsäure-tert-butylamid

Zu 15,0 g (48,5 mmol) (*S*)-4-Formyl-1-(trifluoracetyl)piperazin-2-carbonsäure-*tert-*butylamid in 130 ml Isobutylalkohol wurde eine Lösung von 25,5 g (184,5 mmol) Kaliumcarbonat in 130 ml Wasser gegeben und das Gemisch 1 h auf 60-65 °C erhitzt. Nach der Phasentrennung wurde die wässrige Phase mit 100 ml Isobutylalkohol extrahiert und die vereinigten organischen Phasen eingedampft. Es wurden 14,10 g der rohen (noch lösungsmittelhaltigen) Titelverbindung als hellgelbes Öl erhalten.
- MS: *m/z =*: 213 (M⁺, 1%), 198 (1%), 155 (2%), 113 (100%), 85 (36%), 5.6 (89%).
- ¹H NMR (CDCl₃, 400 MHz) δ =: 8,06 und 8,04 (2 br. s, 1H);
6,86 und 6,47 (2 br. s, 1H);
4,32-4,27 und 3,76-3,61 (3 m, 2H);
3,48-3,41 ("dd", 1H);
3,28-3,15 (m, 2H);
3,07-2,90 (m, 1H);
2,80-2,71 (m, 1H);
1,35 (s, 9H).

### Beispiel 49

### (S)-4-Formylpiperazin-2-carbonsäure-tert-butylamid-Essigsäuresalz (1:1)

Zu 21,58 g (101,2 mmol) (*S*)-4-Formylpiperazin-2-carbonsäure-*tert*-butylamid in 120 ml Tetrahydrofuran wurden 120 ml Essigsäure gegeben und das Gemisch zur Trockne eingedampft. Anschliessend wurden 100 ml Toluol zugegeben und das Gemisch am Rotationsverdampfer eingeengt. Nach der Zugabe von 100 ml 1,4-Dioxan wurde nochmals eingeengt, wobei ein Feststoff ausfiel. Der Rückstand wurde mit 150 ml Ethylacetat aufgeschlämmt und abfiltriert. Nach Waschen mit Ethylacetat und Diethylether und Trocknen wurden 20,10 g (73%) der Titelverbindung als weisser, kristalliner Feststoff erhalten.
- ¹H NMR (DMSO-d₆, 400 MHz) δ =: 8,00 und 7,98 (2 s, 1H);
7,37 und 7,33 (2 br. s, 1H);
4,09-4,03 und 3,68-3,43 (4 "d", 2H);
3,21-3,11 (m, 1H);
3,08-2,99 (m, 1H);
2,96-2,87 (m, 1H);
2,83-2,76 und 2,70-2,61 (2 m, 1 H);
2,59-2,46 (m, 1H);
1,91 (s, 3H);
1,26 (s, 9H).

### Beispiel 50

### (S)-1-(Trifluoracetyl)piperazin-2-carbonsäure-tert-butylamid

Zu 32,0 g (103 mmol) (*S*)-4-Formyl-1-(trifluoracetyl)piperazin-2-carbonsäure-*tert*-butylamid in 200 ml Aceton wurde eine Mischung von 50,5 g (430 mmol) 32%iger Salzsäure und 50 ml Wasser gegeben und 2,5 h auf 60-65 °C erhitzt. Nach dem Abkühlen auf 20 °C wurde mit Triethylamin so neutralisiert, dass die Temperatur unter 30 °C blieb. Das Lösungsmittel wurde abgedampft und 100 ml Wasser und 200 ml Methylisobutylketon zugegeben. Bei 50 °C wurden die Phasen getrennt und die wässrige Phase noch zweimal mit je 100 ml Methylisobutylketon extrahiert. Die vereinigten organischen Extrakte wurden über Magnesiumsulfat gerührt und zur Trockne eingedampft. Es wurden 23,5 g (80%) der Titelverbindung als leicht rötlicher Feststoff erhalten. Umkristallisation aus Toluol/Diisopropylether ergab die Titelverbindung als weisses, kristallines Pulver.
- Schmp.:: 143,5-144,2 °C
- ¹H NMR (CDCl₃, 400 MHz) (Nur die Signale des Hauptisomeren sind angegeben) δ =: 5,88 (br. s, 1H);
4,68-4,65 ("d", 1H);
3,85-3,78 ("d", 1H);
3,54-3,49 ("d", 1H);
3,41-3,32 ("dt", 1H);
3,11-3,06 ("d", 1H);
2,90-2,78 (m, 2H);
2,07 (s, 1H);
1,34 (s, 9H).

### Beispiel 51

### (S)-3-(tert-Butylcarbamoyl)piperazin-1-carbonsäure-tert-butylester [=(S)-4-(tert-Butoxycarbonyl)piperazin-2-carbonsäure-tert-butylamid]

In einem 11 Doppelmantelrührwerk wurde das Reaktionsgemisch aus der asymmetrischen Hydrierung von 50,0 g (162,7 mmol) 4-Formyl-1-trifluoracetyl-1,4,5,6-tetrahydropyrazin-2-carbonsäure-*tert*-butylamid vorgelegt, mit einer Mischung von 64,75 g (567 mmol) 32%iger Salzsäure und 130 g Wasser versetzt und 2 h zum Rückfluss (*ca.* 60 °C) erhitzt. Das Reaktionsgemisch wurde auf 20 °C abgekühlt und so mit 73,76 g (729 mmol) Triethylamin versetzt, dass die Temperatur nicht über 30 °C stieg. Anschliessend gab man innerhalb 1 h bei 30 °C eine Lösung von 31,82 g (145,8 mmol) Di-*tert*-butyl-dicarbonat in 35 g Aceton zu. Nach 15 min Nachreaktionszeit destillierte man das Aceton bei max. 40 °C und 100 mbar ab. Zum Rückstand wurden 300 ml Methanol und eine Lösung von 45,36 g (1,13 mol) Natriumhydroxid in 65 g Wasser gegeben, das Gemisch 2 h zum Rückfluss erhitzt und anschliessend das überschüssige Methanol bei maximal 48 °C und 100 mbar abdestilliert. Danach gab man 100 ml Wasser und 200 ml Methylcyclohexan zu, erhitzte 5 min auf 55 °C und trennte die Phasen. Die wässrige Phase wurde noch zweimal mit je 75ml Methylcyclohexan von 55°C gewaschen und die vereinigten organischen Phasen bei 50-60 °C teilweise eingedampft, wobei die Kristallisation begann. Dann wurde auf 15 °C abgekühlt und die Temperatur 1 h gehalten. Nach einer weiteren Stunde bei 0 °C wurde filtriert und der Rückstand mit 100 ml kaltem Diisopropylether gewaschen. Nach dem Trocknen wurden 35,45 g (77%) der Titelverbindung als weisser, kristalliner Feststoff erhalten.
- ee =: 98,7%
- ¹H NMR (400 MHz, CDCl₃) δ =: 6,75-6,45 (br. s, 1H);
4,13-4,02 (m, 1H);
3,90-3,70 (br. s, 1H);
3,20 (dd, *J=* 3,7, 9,3 Hz, 1H);
3,05-2,80 (m, 3H);
2,80-2,70 (m, 1H);
2,05-1,90 (br. s, 1H);
1,47 (s, 9H);
1,35 (s, 9H).
- ¹³C NMR (100 MHz, CDCl₃) δ =: 170,27;
154,76;
80,11;
58,93;
50,93;
43,40;
44,17;
44,00;
28,75;
28,43.

## Patentansprüche

1. Optisch aktive 2-Piperazincarbonsäurederivate der allgemeinen Formel 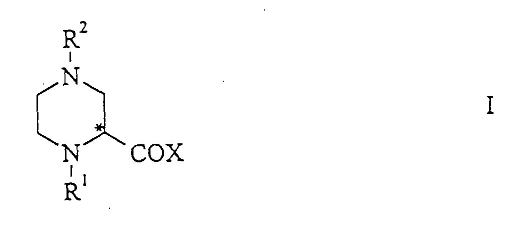 worin R¹ C₁₋₆-Alkanoyl oder C₂₋₆-Perfluoralkanoyl ist, R₂ Wasserstoff, C₁₋₄-Alkyl, gegebenenfalls substituiertes C₁₋₆-Alkanoyl, C₂₋₆-Perfluoralkanoyl, Aroyl, Arylalkyl, C₁₋₆-Alkoxycarbonyl, Aryloxycarbonyl, Carbamoyl oder eine Aminoschutzgruppe bedeutet und X eine C₁₋₆-Alkoxygruppe oder eine Gruppe der Formel -NR³R⁴ ist, worin wiederum
(i) R3 und R4 unabhängig voneinander Wasserstoff, C₁₋₆-Alkyl, C₃₋₆-Cycloalalkyl, Aryl oder eine Aminoschutzgruppe bedeuten oder
(ii) R³ und R⁴ zusammen mit dem Stickstoffatom einen gegebenenfalls substituierten 5- oder 6-gliedrigen gesättigten heterocyclischen Ring bilden oder
(iii) R³ Wasserstoff und R⁴ eine Gruppe der Formel 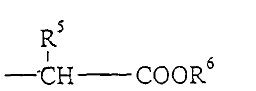 ist, worin R⁵ Wasserstoff oder die Seitengruppe einer Aminosäure und R⁶ Wasserstoff, C₁₋₄-Alkyl oder Aryl ist,
sowie deren Enantiomerengemische.

2. Optisch aktive 2-Piperazincarbonsäurederivate nach Anspruch 1, dadurch gekennzeichnet, dass R¹ Trifluoracetyl ist.

3. Optisch aktive 2-Piperazincarbonsäurederivate der allgemeinen Formel 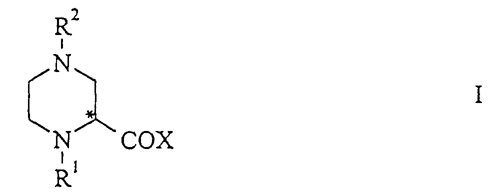 worin R2 C₁₋₆-Alkanoyl oder C₂₋₆-Perfluoralkanoyl ist, R¹ Wasserstoff, C₁₋₄-Alkyl, gegebenenfalls substituiertes C₁₋₆-Alkanoyl, C₂₋₆-Perfluoralkanoyl, Aroyl, Arylalkyl, C₁₋₆-Alkoxycarbonyl, Aryloxycarbonyl, Carbamoyl oder eine Aminoschutzgruppe bedeutet und X die in Anspruch 1 genannte Bedeutung hat, sowie deren Enantiomerengemische.

4. Optisch aktive 2-Piperazincarbonsäurederivate nach Anspruch 3, dadurch gekennzeichnet, dass R² Formyl ist.

5. Verfahren zur Herstellung von optisch aktiven 2-Piperazincarbonsäurederivaten nach Anspruch 1 oder Anspruch 3, dadurch gekennzeichnet, dass ein entsprechendes 1,4,5,6-Tetrahydro-2-pyrazincarbonsäurederivat der allgemeinen Formel 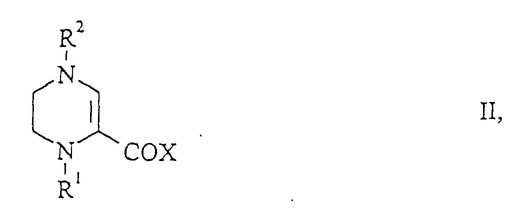 worin R¹, R² und X die in den Ansprüchen 1 bzw. 3 genannte Bedeutung haben, in Gegenwart eines katalytisch wirksamen optisch aktiven Rhodium-, Ruthenium- und/oder Iridiumkomplexes asymmetrisch hydriert wird.

6. Verfahren nach Anspruch 5, dadurch gekennzeichnet, dass als 1,4,5,6-Tetrahydro-2-pyrazincarbonsäurederivat II eine Verbindung eingesetzt wird, in der R¹ und/oder R² jeweils C₁₋₆-Alkanoyl, C₂₋₆-Perfluoralkanoyl, *tert*-Butoxycarbonyl oder Benzyloxycarbonyl ist.

7. Verfahren nach Anspruch 5 oder 6, dadurch gekennzeichnet, dass als 1,4,5,6-Tetrahydro-2-pyrazincarbonsäurederivat II ein Amid eingesetzt wird, in dem X eine Gruppe der Formel -NR³R⁴ ist und R³ und R⁴ die in Anspruch 1 genannten Bedeutungen haben.

8. Verfahren nach Anspruch 7, dadurch gekennzeichnet, dass R³ Wasserstoff und R⁴ eine C₁₋₆-Alkylgruppe ist.

9. Verfahren nach Anspruch 8, dadurch gekennzeichnet, dass R⁴ eine *tert*-Butylgruppe ist.

10. Verfahren nach einem der Ansprüche 5 bis 9, dadurch gekennzeichnet, daß als optisch aktiver Rhodiumkomplex ein aus einem Rh(I)-Komplex und einem optisch aktiven Metallocenylphosphin gebildeter Komplex eingesetzt wird.

11. Verfahren nach Anspruch 10, dadurch gekennzeichnet, dass als optisch aktives Metallocenylphosphin eine Verbindung der allgemeinen Formel 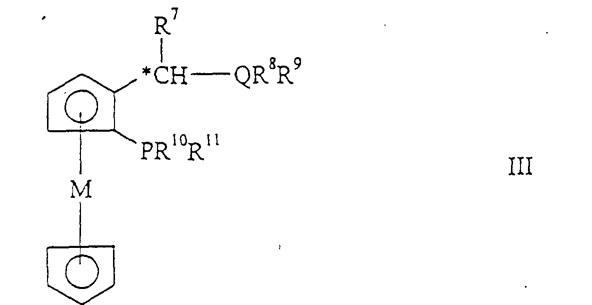 worin M Eisen(II) oder Ruthenium(II), Q Stickstoff oder Phosphor, R⁷ eine C₁₋₄-Alkylgruppe und R⁸ bis R¹¹ unabhängig voneinander C₁₋₈-Alkyl, C₅₋₈-Cydoalkyl, Phenyl oder substituiertes Phenyl bedeuten, eingesetzt wird.

12. Verfahren nach Anspruch 11, dadurch gekennzeichnet, dass M Eisen(II) ist.

13. Verfahren nach Anspruch 11 oder 12, dadurch gekennzeichnet, dass Q Phosphor ist.

14. Verfahren nach einem der Ansprüche 11 bis 13, dadurch gekennzeichnet, dass R⁷ Methyl ist und R⁸ und R⁹ gleich sind und *tert*-Butyl oder Cyclohexyl bedeuten.

15. Verfahren nach einem der Ansprüche 11 bis 14, dadurch gekennzeichnet, dass als Rh(I)-Komplex ein neutraler Komplex der allgemeinen Formel
[Rh(L)A]₂ IV
worin L ein C₄₋₁₂-Dien oder zwei C₂₋₁₂-Alkenmoleküle bedeutet und A Chlor, Brom oder lod ist, eingesetzt wird.

16. Verfahren nach einem der Ansprüche 11 bis 14, dadurch gekennzeichnet, dass als Rh(I)-Komplex ein kationischer Komplex der allgemeinen Formel
[Rh(L)₂]⁺B⁻ V
worin L ein C₄₋₁₂-Dien oder zwei C₂₋₁₂-Alkenmoleküle bedeutet und B⁻ das Anion einer Sauerstoffsäure oder einer Komplexsäure ist, eingesetzt wird.

17. Verfahren nach Anspruch 15 oder 16, dadurch gekennzeichnet, dass L Norbornadien oder 1,5-Cyclooctadien bedeutet.
